# DEMANDE DE BREVET EUROPEEN

(11) **EP 1 120 126 A2**
(43) Date de publication de la demande: **01.08.2001**
(21) Numéro de dépôt: 01400054.1
(22) Date de dépôt: 10.01.2001
(51) Int. Cl.: A61M 16/01

(54) **Ventilateur d'anesthésie avec commutation automatique de circuit**

(30) Priorité: 19.01.2000 FR 0000636
(71) Demandeur: TAEMA, F-92182 Antony Cédex (FR)
(72) Inventeur: Masson, Jean-Philippe, 92370 Chaville (FR); Kitten, Sébastien, 91160 Saulx les Charteux (FR)
(74) Mandataire: Pittis, Olivier

(57) **Abrégé**

L'invention concerne un dispositif de distribution de gaz à un patient, notamment de gaz anesthésiants, avec réinhalation du gaz expiré.
Il comprend :
- une première conduite (10) de circulation de gaz, un dispositif de commutation (12) comprenant une entrée (14) raccordée à ladite première conduite (10) et une première et une deuxième sortie (16, 18), une deuxième portion de conduite (30) de circulation du gaz , un ensemble amovible de filtrage de gaz (20), ledit ensemble comprenant une entrée de gaz (24) reliable à la première sortie du dispositif de commutation et une sortie de gaz (28) reliable à ladite deuxième conduite, une conduite de dérivation (34) dont une première extrémité est reliée à la deuxième sortie (18) du dispositif de commutation et dont l'autre extrémité est reliée à ladite deuxième conduite, des moyens de détection (36) pour détecter la présence dudit ensemble de filtrage dans le circuit ; et des moyens de commande (38) du dispositif de commutation (12) pour faire prendre audit dispositif de commutation un premier état dans lequel son entrée (14) est reliée à sa première sortie (16), et pour, en l'absence d'information de détection, faire prendre audit dispositif de commutation un deuxième état dans lequel son entrée (14) est reliée à sa deuxième sortie (18).

## Description

La présente invention a pour objet un dispositif de distribution de gaz à un patient et notamment de gaz anesthésiants avec réinhalation du gaz expiré par le patient.

Lors d'une intervention chirurgicale sous anesthésie, il est nécessaire de fournir au patient un mélange de gaz qui comprend le plus souvent de l'oxygène, un gaz anesthésiant, et le plus souvent également de l'air.

Dans un certain nombre de cas, par exemple dans le cas décrit dans la demande de brevet européen 835 672, le dispositif de distribution, au patient, du gaz est en circuit fermé, c'est-à-dire que le gaz expiré par le patient est réinhalé par celui-ci. Une telle installation présente pour l'utilisateur l'intérêt de limiter la quantité de gaz puisque celle-ci se réduit après un remplissage initial à la compensation des pertes dues aux fuites et à la consommation des gaz par le patient. On comprend que dans une telle installation il est bien sûre nécessaire de procéder au filtrage du gaz recyclé afin d'en extraire notamment le dioxyde de carbone.

Le plus souvent, le média servant à la filtration du dioxyde de carbone doit être changé, éventuellement au cours d'anesthésie lorsque l'intervention chirurgicale est relativement longue. Cela nécessite généralement une dépose du conteneur rempli du média filtrant qui est monté en série dans la ligne de débit des gaz administrés ou provenant du patient. Le retrait de ce conteneur peut être traité de différentes manières.

Selon que le système de filtrage des gaz est situé sur la branche inspiration ou sur la branche expiration du circuit avec réinhalation, plusieurs solutions ont déjà été mises en pratique.

Dans le cas où le système d'épuration et de filtrage est sur la branche inspiratoire, on peut soit procéder à l'arrêt complet de l'insufflation de gaz par ouverture du circuit en vue du changement du matériau de filtrage, soit utiliser un organe mécanique qui permet, par actionnement manuel, avant le retrait du conteneur rempli du matériau de filtrage, de créer un circuit fermé avec réinhalation du gaz mais ce circuit est dépourvu de filtrage.

L'ensemble des solutions exposées ci-dessus présente des risques ou désagréments pour l'utilisateur. Dans le cas de la première solution, le patient n'est pas ventilé durant le changement du média de filtrage et en cas de bris de l'enceinte contenant ce média la machine est inutilisable. Enfin, l'ouverture du circuit provoque une modification dans les concentrations de gaz préalablement établies dans ce circuit ainsi qu'un échappement, dans l'atmosphère, des gaz anesthésiants qui peuvent polluer le bloc opératoire.

Dans le cas de la deuxième solution, l'utilisateur doit effectuer une manipulation supplémentaire avant et après le retrait du conteneur rempli avec le matériau de filtrage pour, dans un premier temps, continuer la ventilation circuit fermé avec réinhalation mais sans épuration du gaz avant de revenir à un circuit fermé avec réinhalation et avec épuration du gaz par filtrage.

Dans le cas où le système d'épuration est placé sur la branche expiratoire les deux solutions envisageables sont les suivantes :
- soit on utilise un système manuel assurant le maintien du circuit fermé mais sans que celui-ci comporte de système de filtrage lorsque celui-ci est démonté. On est confronté aux mêmes difficultés que dans le cas de la branche inspiratoire.
- soit l'enlèvement du système de filtrage entraîne l'ouverture de la branche expiratoire avec maintien de la ventilation du patient. Le patient est toujours raccordé à la machine pour l'insufflation mais les gaz expirés sont éliminés en totalité sans aucun recyclage. L'utilisation doit augmenter considérablement la quantité de gaz délivrée par la machine.

Dans ce dernier cas, l'ouverture du circuit présente les mêmes conséquences que la première solution en ce qui concerne les concentrations établies des gaz dans le circuit et la pollution éventuelle du bloc opératoire. De plus, il demande à l'opérateur d'augmenter ses débits de gaz pour fonctionner en circuit sans réinhalation.

La présente invention a pour objet un dispositif de distribution de gaz à un patient, notamment de gaz anesthésiants, avec réinhalation du gaz expiré qui permet d'éviter les inconvénients mentionnés ci-dessus en assurant en permanence la ventilation du patient et en ne nécessitant aucune opération particulière de la part de l'opérateur lors de l'enlèvement du conteneur rempli du matériau filtrant en vue de son changement.

Pour atteindre ce but selon l'invention, le dispositif de distribution de gaz à un patient notamment de gaz anesthésiant avec réinhalation du gaz expiré comprend :
- une première portion de conduite de circulation de gaz,
- un dispositif de commutation comprenant une entrée raccordée à ladite première portion de conduite et une première et une deuxième sortie,
- une deuxième portion de conduite de circulation du gaz présentant deux entrées,
- un ensemble amovible de filtrage de gaz, ledit ensemble comprenant une entrée de gaz reliable à la première sortie du dispositif de commutation et une sortie de gaz reliable à une entrée de ladite deuxième portion de conduite,
- une conduite de dérivation dont une première extrémité est reliée à la deuxième sortie du dispositif de commutation et dont l'autre extrémité est reliée à la deuxième entrée de ladite deuxième portion de conduite,
- des moyens de détection pour détecter la présence dudit ensemble de filtrage dans le circuit et fournir une information de détection en cas de présence dudit ensemble ; et
- des moyens de commande du dispositif de commutation pour, en réponse à l'information de détection fournie par les moyens de détection, faire prendre audit dispositif de commutation un premier état dans lequel son entrée est reliée à sa première sortie, et pour, en l'absence d'information de détection, faire prendre audit dispositif de commutation un deuxième état dans lequel son entrée est reliée à sa deuxième sortie.

On comprend que grâce au dispositif selon l'invention, lorsqu'il y a lieu de procéder à l'enlèvement de l'ensemble de filtrage du circuit de ventilation, cette situation est détectée automatiquement et le dispositif de commutation est commandé également automatiquement pour assurer la réinhalation par le patient du gaz expiré par l'intermédiaire de la conduite de dérivation.

En ce qui concerne les moyens de détection et de commande du dispositif de commutation ils peuvent être mécaniques électriques, ou de toute nature convenable.

Selon un mode préféré de mise en oeuvre correspondant à une détection et à une commande mécanique, le dispositif se caractérise en ce que le dispositif de commutation comprend une enceinte munie de ladite entrée de gaz et desdites première et deuxième sorties de gaz, un obturateur mobile à l'intérieur de ladite enceinte, apte à obturer ladite première sortie ou ladite deuxième sortie et des moyens de rappel de l'obturateur dans une première position correspondant à l'obturation de ladite première sortie, et en ce que les moyens de détection et de commande comprennent un organe mécanique lié à l'entrée dudit ensemble de filtrage, ledit organe mécanique étant apte, lorsque ledit ensemble de filtrage est présent, à amener ledit obturateur dans une deuxième position où il obture ladite deuxième sortie.

On comprend que selon ce mode de réalisation, la détection et la commande du commutateur est très simple et très fiable puisque c'est directement un organe mécanique qui, à la suite de l'enlèvement de l'ensemble de filtrage, provoque le changement d'état du commutateur pour que sa sortie soit reliée à la conduite de dérivation.

D'autres caractéristiques et avantages de l'invention apparaîtront mieux à la lecture de la description qui suit de plusieurs modes de réalisation de l'invention donnés à titre d'exemple non limitatif. La description se réfère aux figures annexées sur lesquelles :
- la figure 1 est un schéma de principe montrant l'ensemble du dispositif ;
- la figure 2 est une vue partielle montrant un exemple de réalisation de la détection de la présence de l'ensemble de filtrage par des moyens mécaniques ; et
- la figure 3 est une vue partielle montrant un système de détection électrique.

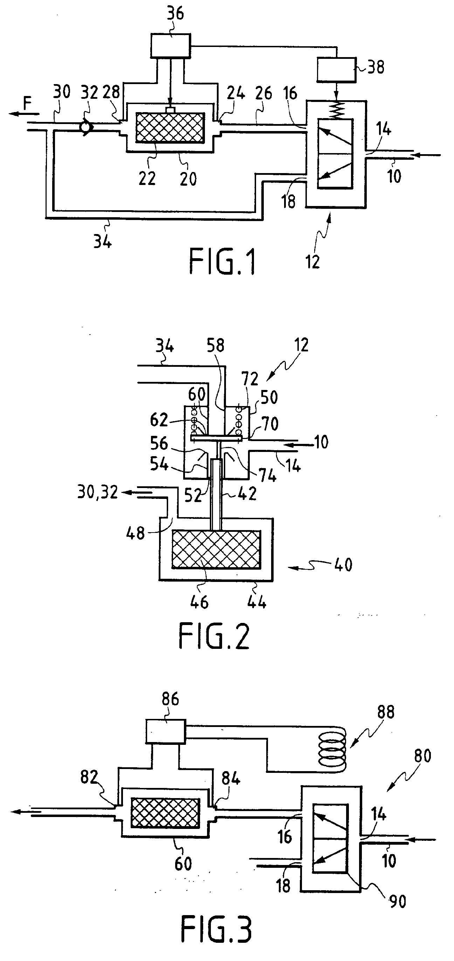

En se référant tout d'abord à la figure 1 on va décrire de façon simplifiée l'ensemble du dispositif de distribution de gaz à un patient.

Sur cette figure on a représenté une première portion de conduite 10 qui achemine le gaz par exemple le gaz anesthésiant. La conduite 10 est raccordée à un commutateur de fluide 12 et plus précisément à son entrée unique 14. Le commutateur 12 a pour fonction de raccorder l'entrée 14 soit à une première sortie 16, soit à une deuxième sortie 18. La première sortie 16 est raccordée à un conteneur 20 qui contient un matériau de filtrage 22 du gaz expiré par le patient afin d'en retenir le dioxyde de carbone. Le conteneur de filtrage 20 comporte une entrée de gaz 24 raccordée à la sortie 16 par un élément de conduite 26. Le raccordement entre la conduite 26 et l'entrée 24 du conteneur 20 est amovible pour permettre, comme on l'expliquera ultérieurement, le démontage du conteneur de filtrage par rapport à l'installation en vue de procéder au changement du matériau de filtrage 22. La sortie de gaz 28 du conteneur 20 est raccordée à une première entrée 30a d'une deuxième portion de conduite 30 de circulation du gaz par l'intermédiaire d'un clapet anti-retour 32 qui n'autorise la circulation du gaz que dans le sens indiqué par la flèche F. L'installation comporte également une conduite de dérivation 34 qui raccorde directement la deuxième sortie 18 du commutateur 12 à la deuxième entrée 30b de la deuxième portion de conduite 30 en aval du clapet anti-retour 32.

L'installation comporte également un circuit de détection 36 qui permet de détecter la présence effective du conteneur 20 dans le circuit. Les moyens de détection 36 sont raccordés à des moyens de commande 38 du commutateur 12.

En fonctionnement normal le conteneur de filtrage 20 est présent dans le circuit. Cette présence, constatée par les moyens 36, est transmise au moyen de commande 38 du commutateur qui occupe un premier état dans lequel son entrée 14 est reliée à sa sortie 16. Ainsi, le débit de gaz traverse le conteneur de filtrage et en sort purifié pour pénétrer dans la deuxième portion de conduite 30.

Lorsque l'utilisateur, par exemple l'anesthésiste, veut procéder au changement du conteneur de filtrage, celui-ci désolidarise le conteneur des conduites 26 et 30. Cette situation est automatiquement détectée par les moyens de détection 36 et elle est transmise au moyen de commande 38 du commutateur 12. Celui-ci prend son deuxième état qui met en relation l'entrée 14 avec la sortie 18. Ainsi, le nouveau circuit de ventilation du patient est constitué par la portion de conduite 10, la conduite de dérivation 34 et la portion de conduite 30.

Lorsque l'utilisateur veut procéder à la mise en place du nouveau conteneur 20 de filtrage celui-ci est reconnecté aux conduites 26 et 30. La présence d'un nouveau conteneur de filtrage est détectée en 36 et les moyens de commande 38 font passer le commutateur 12 dans son premier état qui remet le circuit de ventilation dans son état de fonctionnement normal c'est-à-dire avec le filtrage de l'air expiré.

Ainsi qu'on l'a déjà expliqué les moyens de détection 36 de la présence de l'ensemble de filtrage et les moyens 38 de commande de l'état du commutateur 12 peuvent être, de préférence, électriques ou mécaniques. En se référant à la figure 2 on va décrire un mode de réalisation mécanique de ces deux ensembles.

Sur cette figure 2 on a référencé 40 le conteneur de filtrage et 12 le commutateur mécanique. Le conteneur 40 qui est constitué par une enceinte 44 contenant le matériau de filtrage 46 comporte une sortie 48 raccordée à la deuxième portion de conduite de gaz 30. L'entrée de gaz du conteneur 40 est constituée par une cheminée 42 qui fait saillie hors de ce conteneur 40.

Le commutateur mécanique 12 comprend une enceinte 50 munie d'une première ouverture 52 prolongée intérieurement par un manchon 54 se terminant par un siège de clapet 56. Le manchon 54 est configuré de telle manière qu'il puisse recevoir l'extrémité de la cheminée 42 de façon étanche. L'enceinte 50 du commutateur comporte une deuxième ouverture 58 prolongée par un manchon interne 60 formant un deuxième siège de clapet 62 en regard du premier siège de clapet 56. Le manchon 58 est raccordé à la conduite de dérivation 34. Enfin, l'enceinte 50 du commutateur comporte l'entrée de gaz 14 raccordée à la conduite de gaz 10. Le commutateur 12 comporte également un obturateur mobile 70 qui peut prendre deux positions en venant s'appliquer soit sur le siège 56, soit sur le siège 62. Un ressort de rappel 72 tend à maintenir l'obturateur 70 sur le siège 56. Des moyens de guidage non représentés sont associés à l'obturateur mobile 70.

Dans ce mode de réalisation, l'extrémité de la cheminée 42 du conteneur de filtrage 40 est prolongée par une tige 74 qui, lorsque le conteneur 40 est engagé dans le manchon 54, agit sur l'obturateur 70 pour maintenir celui-ci plaqué contre le siège 62 en comprimant le ressort de rappel 72. Dans cette position, le gaz arrive par l'entrée 14 dans le commutateur 12 et passe dans la cheminée 42 du conteneur de filtrage puisque le manchon 60 est obturé par l'obturateur 70. En revanche, lorsque le conteneur de filtrage est enlevé, le ressort de rappel 72 applique l'obturateur 70 sur le siège 56 et libère ainsi l'ouverture du manchon 60. Le gaz pénétrant dans le commutateur 12 par l'entrée 14 en ressort par le manchon 60 et la conduite de dérivation 34.

Cette solution mécanique comporte l'intérêt de présenter une très grande fiabilité puisque ce sont directement des organes mécaniques qui provoquent à la fois la détection de la présence du conteneur de filtrage, la transmission de l'information de détection et la commande du commutateur dans son état correspondant.

On comprend que dans ce mode de réalisation, l'information de détection consiste dans la détection de la présence ou de l'absence de la tige 74 agissant sur l'obturation 70.

La figure 3 illustre un système de détection et de commande du commutateur de type électrique.

Des capteurs électriques 82 et 84 sont montés dans les deux zones de raccordement du conteneur de filtrage 60 avec les conduites. Lorsqu'un des raccordements est démonté, le capteur correspondant transmet un signal au circuit de traitement 86.

Le circuit de traitement 86 applique à une bobine de commande 88 un courant convenable pour amener le tiroir 90 du commutateur 80 dans une position où il met en communication l'entrée 14 avec la sortie 18. Lorsque l'enceinte de filtrage 60 est à nouveau branchée sur le deux conduites, les capteurs 82 et 84 émettent vers le circuit de traitement 86 un signal. Le circuit 86 applique alors à la bobine de commande 88 un courant électrique convenable pour amener le tiroir 90 dans une position où il met en communication l'entrée 14 du commutateur avec sa sortie 16.

L'ensemble de filtrage 20 ainsi que le commutateur 12 et la conduite de dérivation 34 peuvent être montés aussi bien dans la branche inspiratoire que dans la branche expiratoire, les portions de conduite 10 et 30 constituant alors soit la conduite d'inspiration pour amener au patient le mélange de gaz, soit la conduite d'expiration pour recueillir le mélange gazeux expiré par le patient et le recycler.

Le dispositif de distribution de gaz peut constituer soit un circuit fermé d'anesthésie, soit un circuit semi-fermé d'anesthésie.

## Revendications

1. Dispositif de distribution de gaz à un patient, notamment de gaz anesthésiants, avec réinhalation du gaz expiré comprenant :
- une première portion de conduite (10, 12) de circulation de gaz,
- un dispositif de commutation comprenant une entrée (14) raccordée à ladite première portion de conduite (10) et une première et une deuxième sortie (16, 18),
- une deuxième portion de conduite (30) de circulation du gaz présentant deux entrées (30a, 30b),
- un ensemble amovible de filtrage de gaz (20), ledit ensemble comprenant une entrée de gaz (24) reliable à la première sortie du dispositif de commutation et une sortie de gaz (28) reliable à une entrée de ladite deuxième portion de conduite,
- une conduite de dérivation (34) dont une première extrémité est reliée à la deuxième sortie (18) du dispositif de commutation et dont l'autre extrémité est reliée à la deuxième entrée (30b) de ladite deuxième portion de conduite,
- des moyens de détection (36) pour détecter la présence dudit ensemble de filtrage dans le circuit et fournir une information de détection en cas de présence dudit ensemble ; et
- des moyens de commande (38) du dispositif de commutation (12) pour, en réponse à l'information de détection fournie par les moyens de détection, faire prendre audit dispositif de commutation un premier état dans lequel son entrée (14) est reliée à sa première sortie (16), et pour, en l'absence d'information de détection, faire prendre audit dispositif de commutation un deuxième état dans lequel son entrée (14) est reliée à sa deuxième sortie (18).

2. Dispositif selon la revendication 1, caractérisé en ce que ladite deuxième portion de conduite (30) est munie de moyens anti-retour (32) et en ce que la deuxième extrémité de la conduite de dérivation (34) est raccordée à l'entrée de ladite deuxième portion de conduite (30) en aval desdits moyens anti-retour.

3. Dispositif selon l'une quelconque des revendications 1 et 2, caractérisé en ce que lesdits moyens de détection (82, 84, 86) et lesdits moyens de commande (88) du dispositif de commutation (12) sont électriques.

4. Dispositif selon l'une quelconque des revendications 1 et 2, caractérisé en ce que lesdits moyens de détection et lesdits moyens de commande du dispositif de commutation sont mécaniques.

5. Dispositif selon la revendication 4, caractérisé en ce que ledit dispositif de commutation (12) comprend une enceinte (50) munie de ladite entrée de gaz (14) et desdites première (52) et deuxième (58) sorties de gaz, un obturateur mobile (70) à l'intérieur de ladite enceinte, apte à obturer ladite première sortie ou ladite deuxième sortie et des moyens de rappel (72) de l'obturateur dans une première position correspondant à l'obturation de ladite première sortie, et en ce que les moyens de détection et de commande comprennent un organe mécanique (74) lié à l'entrée (42) dudit ensemble de filtrage (40), ledit organe mécanique étant apte, lorsque ledit ensemble de filtrage est présent, à amener ledit obturateur (70) dans une deuxième position où il obture ladite deuxième sortie.

6. Dispositif selon l'une quelconque des revendications 1 à 5, caractérisé en ce que lesdites première (10) et deuxième (30) portions de conduite constituent la branche inspiration dudit dispositif de distribution de gaz.

7. Dispositif selon l'une quelconque des revendications 1 à 5, caractérisé en ce que lesdites première (10) et deuxième (30) portions de conduite constituent la branche expiration dudit dispositif de distribution de gaz.

8. Dispositif selon l'une quelconque des revendications 1 à 7, caractérisé en ce que ledit gaz distribué est un gaz ou un mélange de gaz anesthésiant et en ce que ledit dispositif de distribution est du type en circuit fermé.

9. Dispositif selon l'une quelconque des revendications 1 à 7, caractérisé en ce que ledit gaz distribué est un gaz ou un mélange de gaz anesthésiant et en ce que ledit dispositif de distribution est du type en circuit semi-fermé.
